# EUROPÄISCHE PATENTSCHRIFT

(11) **EP 3 720 539 B1**
(45) Veröffentlichungstag und Bekanntmachung des Hinweises auf die Patenterteilung: **24.07.2024**
(21) Anmeldenummer: 18822012.3
(22) Anmeldetag: 04.12.2018
(51) Int. Cl.: A61M 35/00, A61J 1/06, B67B 7/92

(54) **VORRICHTUNG ZUM AUSTRAGEN EINES FLIESSFÄHIGEN STOFFES**
DEVICE FOR DISCHARGING A POURABLE SUBSTANCE
DISPOSITIF DESTINÉ À DISTRIBUER UNE MATIÈRE FLUIDE

(30) Priorität: 05.12.2017 DE 102017128918; 26.06.2018 DE 102018115344
(43) Veröffentlichungstag der Anmeldung: 14.10.2020
(73) Patentinhaber: Hiemer, Andreas, 26871 Papenburg (DE); Sògaro, Alberto C., 61348 Bad Homburg (DE); Seitz, Sebastian, 91602 Dürrwangen (DE)
(72) Erfinder: Hiemer, Andreas, 26871 Papenburg (DE); Sògaro, Alberto C., 61348 Bad Homburg (DE); Seitz, Sebastian, 91602 Dürrwangen (DE)
(74) Vertreter: advotec.
(86) Internationale Anmeldenummer: PCT/EP2018/083527
(87) Internationale Veröffentlichungsnummer: WO 2019/110605

(56) Entgegenhaltungen:
- WO-A1-2015/042021
- WO-A1-94/06690
- WO-A1-99/09932
- US-A- 3 614 245
- US-A1- 2002 076 255
- US-A1- 2009 152 296

## Beschreibung

Die Erfindung betrifft eine Vorrichtung zum Austragen eines fließfähigen Stoffes, insbesondere zum Applizieren eines medizinischen Stoffes, pharmazeutischen Stoffes, eines Nahrungsergänzungsmittels oder eines Kosmetikums.

Aus der Praxis ist eine Vorrichtung mit einer Innenhülse und einer Außenhülse bekannt, in der eine zerbrechbare Kapsel angeordnet ist und die an einer Stirnseite mit einer Austragöffnung versehen ist. In der Kapsel ist ein fließfähiger Stoff, wie beispielsweise ein Klebstoff, enthalten. Vor einem Applizieren des fließfähigen Stoffes durch die Austragöffnung wird die Kapsel mittels einer in axialer Richtung wirkenden Innenhülse zerbrochen, so dass der fließfähige Stoff durch die Austragöffnung fließt.

Mit der bekannten Vorrichtung ist ein definiertes Applizieren bzw. Pipettieren des fließfähigen Stoffes nicht möglich. Auch ist die Kapsel in der Vorrichtung nicht positionsgesichert.

Aus Druckschrift US 2002/076255 A1 ist ein Applikator bekannt, umfassend einen hohlen Körper, in dem eine Kapsel angeordnet ist, eine Aktivierungseinrichtung und eine Austrageöffnung.

Der Erfindung liegt daher die Aufgabe zugrunde, eine Vorrichtung zum Austragen eines fließfähigen Stoffes zu schaffen, mittels der ein zunächst in einer zerbrechbaren Kapsel beinhalteter fließfähiger Stoff definiert applizierbar bzw. ausbringbar ist und eine Position der Kapsel in der Vorrichtung gesichert bzw. festgelegt ist.

Diese Aufgabe ist erfindungsgemäß durch die Vorrichtung mit den Merkmalen des Anspruchs 1 gelöst.

Erfindungsgemäß wird also eine Vorrichtung mit einem Körper, wie beispielsweise einer Applikatorhülse, zum Austragen eines fließfähigen Stoffes, insbesondere zum Applizieren eines medizinischen Stoffes, pharmazeutischen Stoffes, eines Nahrungsergänzungsmittels oder eines Kosmetikums vorgeschlagen. Der Körper hat an einem ersten Ende eine Austragsöffnung und bildet mit einem Wandbereich einen Aufnahmeraum aus, in dem eine zerbrechbare Kapsel, die den fließfähigen Stoff enthält, positioniert ist. An einem zweiten Ende des Körpers ist eine Aktivierungseinrichtung angeordnet. Die Kapsel hat an ihren beiden Enden jeweils eine Spitze, wobei durch Betätigung der Aktivierungseinrichtung beide Spitzen abbrechbar sind, so dass der fließfähige Stoff definiert ausgebracht bzw. appliziert werden kann.

Bei einer zweckmäßigen Ausführungsform besteht die Kapsel aus Glas. Glas weist den Vorteil auf, dass es gegenüber einer Vielzahl von Stoffen chemisch resistent ist und demnach insbesondere zur Aufnahme von medizinischen oder pharmazeutischen Stoffen gut geeignet ist.

Alternativ kann die Kapsel aus einem zerbrechbaren gegenüber dem fließfähigen Stoff chemisch resistenten Kunststoff gebildet sein.

Bei der erfindungsgemässen Ausführungsform ist die Kapsel an ihren beiden Enden jeweils mit einer Sollbruchstelle versehen, so dass die beiden Enden jeweils definiert von der Kapsel abgebrochen werden können.

Bei einer vorteilhaften Ausführungsform ist die Kapsel durch die Aktivierungseinrichtung, insbesondere durch einen Vorsprung an dieser und/oder durch eine entsprechende Ausgestaltung des Körpers der Vorrichtung selbst, in dem Aufnahmeraum des Körpers positioniert bzw. positionsgesichert. Hierdurch ist sichergestellt, dass die Spitze(n) der Kapsel korrekt abgebrochen wird/werden und nach Entfernen der Spitze(n) der fließfähige Stoff durch die Austrageöffnung ausbringbar ist.

Bei einer alternativen Ausführungsform hat der Körper an dem ersten Ende einen Endabschnitt, der insbesondere als eine Verengung bzw. Verjüngung ausgestaltet ist, durch den der fließfähige Stoff definiert ausgetragen bzw. appliziert werden kann. Auch kann die erste Spitze der Kapsel in dem Endabschnitt angeordnet sein, wodurch die Kapsel räumlich in dem Körper positioniert ist und ein Nutzer die Spitze ohne direkten Kontakt mit dieser abbrechen kann.

Bei einer vorteilhaften Ausführungsform ist der Endabschnitt aus einem elastischen, stauchbaren oder brechbaren Material. Durch eine Deformation, ein Stauchen oder ein Brechen des Endabschnitts wird die in dem Endabschnitt angeordnete Spitze der Kapsel abgebrochen, so dass der in der Kapsel beinhaltete fließfähige Stoff tröpfchenweise bzw. definiert appliziert werden kann.

Der Endabschnitt kann ein Kippverschluss oder ein Luer-System sein und/oder an dem dem Körper abgewandten Ende einen Löffel ausbilden. Auch kann der Endabschnitt Greifelemente haben, wodurch ein Nutzer den Endabschnitt und somit die erste Spitze auf einfache Weise entfernen kann.

Bei einer alternativen Ausführungsform ist an der Austrageöffnung ein Applikator aus saugfähigem Material angeordnet. Der Applikator nimmt nach einer Aktivierung der Vorrichtung bzw. nach einem Entfernen mindestens einer Spitze der Kapsel das aus der Kapsel fließende Material auf bzw. saugt sich mit diesem voll. Somit kann der fließfähige Stoff auf der anderen Seite des Applikators definiert appliziert werden.

Die Form des Applikators kann insbesondere an der Austrageseite an einen jeweiligen Anwendungsbereich angepasst werden, wodurch eine Nutzerfreundlichkeit verbessert ist.

Die Aktivierungseinrichtung ist beispielsweise ein Kolben, eine Vakuum-Kammer oder besteht aus einem flexiblen Material, wie einer Gummikappe.

Insbesondere kann bei einer als Kolben ausgestalteten Aktivierungseinrichtung die Aktivierungseinrichtung an einer der Kapsel zugewandten Seite eine Abschrägung bzw. eine abgeschrägte Kante haben, sodass bei einem axialen Verschieben der Aktivierungseinrichtung in Richtung der Kapsel die Spitze der Kapsel derart mit einer Kraft beaufschlagt wird, dass die zweite Spitze definiert abbricht, das heißt, dass ein Risiko einer Beschädigung der Kapsel reduziert ist.

Besteht die Aktivierungseinrichtung aus dem flexiblen Material, kann diese mittels eines Fixierelements an dem Körper bzw. der Applikatorhülse angebunden sein. Hierzu weist das Fixierelement beispielsweise Zähne auf, die in korrespondierende Ausnehmungen eingreifen und dort verrasten, oder es hat ein Gewinde, das mit einem an dem Körper ausgebildeten korrespondierenden Gegengewinde in Eingriff ist.

Bei einer vorteilhaften Ausführungsform wird die Aktivierungseinrichtung durch ein axiales Verschieben, ein Umbiegen oder ein Zusammendrückender betätigt, so dass die zweite Spitze oder die erste und die zweite Spitze der Kapsel abgebrochen werden und der fließfähigen Stoff der Kapsel applizierbar bzw. ausbringbar ist.

Insbesondere können bei einer derartigen, beispielsweise als Kolben ausgestalteten Aktivierungseinrichtung durch das axiale Verschieben die erste und die zweite Spitze der Kapsel abgebrochen werden, so dass die Vorrichtung mit einer Hand betätigbar ist.

Bei einer alternativen Ausführungsform hat der Körper eine Rampe, auf die der Kolben bei Betätigen der Aktivierungseinrichtung auffährt. Hierdurch ist eine Relativbewegung des Kolbens bezogen auf den Körper begrenzt, so dass eine Beschädigung der Kapsel durch den Kolben ausgeschlossen ist.

Vorzugsweise ist die Rampe an dem Wandbereich des Körpers oder an dem zweiten Ende des Körpers ausgebildet.

Bei einer vorteilhaften Ausführungsform hat die Aktivierungseinrichtung einen Zapfen und der Körper einen mit dem Zapfen korrespondierenden Schlitz. Der Zapfen der Aktivierungseinrichtung ist in dem Schlitz des Körpers positioniert, wodurch ein Verschiebe- bzw. Rotationsweg des Kolbens relativ zum Körper begrenzt ist.

Hierdurch wird auf einfache Weise sichergestellt, dass bei dem Betätigen der Aktivierungseinrichtung die zweite Spitze der Kapsel abbricht, ein Kapselkörper jedoch unbeschädigt bleibt.

Bei einer bevorzugten Ausführungsform ist zwischen dem Körper und der Aktivierungseinrichtung ein Rückstellelement angeordnet, das die Aktivierungseinrichtung nach dem Betätigen in ihre Ausgangsstellung (zurück-)bringt. Hierdurch kann, insbesondere, wenn die Aktivierungseinrichtung ein Kolben ist, zum Ausbringen des fließfähigen Stoffes eine sogenannte Pump-Bewegung realisiert werden.

Vorzugsweise ist an dem Körper eine Greifhilfe angeordnet bzw. von dem Körper ausgebildet, die die Bedienfreundlichkeit erhöht.

Bei einer alternativen Ausführungsform weist der Körper einen deformierbaren Bereich auf, mittels welchem der fließfähigen Stoff definiert ausgetragen bzw. appliziert werden kann.

Bei einer vorteilhaften Ausführungsform ist der Körper eine Tube und die Aktivierungseinrichtung eine Verschlussnaht, insbesondere eine Schweißnaht, die an dem der Austrageöffnung abgewandten Ende des Körpers angeordnet ist. Die zweite Spitze der Kapsel oder ein Teil dieser befindet sich in der Verschlussnaht, so dass durch eine Betätigung der Aktivierungseinrichtung, insbesondere ein Umknicken der Verschlussnaht die zweite Spitze der Kapsel abbricht, ohne dass ein Nutzer mit der Kapsel selbst in Kontakt kommt.

An dem vorderen Ende der Tube kann ein Knickverschluss angeordnet sein, in der sich die erste Spitze der Kapsel befindet, so dass durch ein Abbrechen des Knickverschlusses auch die erste Spitze der Kapsel abbricht.

Insbesondere kann die Verschluss- bzw. Schweißnaht derart ausgestaltet sein, dass der Nutzer eine handelsübliche Tube wahrnimmt.

Auch kann die Tube aus einem steifen Material sein, so dass sie nicht deformierbar ist.

Bei einer vorteilhaften Ausführungsform ist die Aktivierungseinrichtung mit einem Kennzeichen, wie beispielsweise einem Schriftzeichen, versehen, das den Nutzer auf eine korrekte Aktivierung hinweist.

Bei einer alternativen Ausführungsform bildet der Körper zwei oder mehr Aufnahmeräume aus, in denen jeweils eine Kapsel angeordnet ist, so dass mehrere Spitzen von Kapseln gleichzeitig mittels Betätigung einer Aktivierungseinrichtung abgebrochen werden können und somit mehrere fließfähige Stoffe appliziert werden können, ohne dass sich diese zuvor vermischen bzw. vermengen.

Ausführungsbeispiele einer Vorrichtung nach der Erfindung sind in der Zeichnung schematisch vereinfach dargestellt und werden in der nachfolgenden Beschreibung näher erläutert. Es zeigt:
- Fig. 1: eine perspektivische Ansicht einer in einer Vorrichtung nach der Erfindung angeordneten Kapsel;
- Fig. 2: einen Schnitt durch eine erste Ausführungsform einer Vorrichtung nach der Erfindung;
- Fig. 3: eine perspektivische Ansicht einer alternativen Ausführungsform der Vorrichtung nach Figur 2;
- Fig. 4: eine Seitenansicht einer alternativen Ausführungsform der Vorrichtung nach Figur 2;
- Fig. 5: eine Seitenansicht einer alternative Ausführungsform der Vorrichtung nach Figur 4;
- Fig. 6: eine perspektivische Ansicht einer alternativen Ausführungsform der Vorrichtung nach Figur 2
- Fig. 7: einen Schnitt durch die Vorrichtung nach Figur 6;
- Fig. 8: eine perspektivische Ansicht einer alternativen Ausführungsform einer Vorrichtung nach der Erfindung;
- Fig. 9: einen Schnitt durch die Vorrichtung nach Figur 8;
- Fig. 10: eine perspektivische Ansicht einer alternativen Ausführungsform der Vorrichtung nach Figur 8;
- Fig. 11: eine perspektivische Ansicht einer alternativen Ausführungsform der Vorrichtung nach Figur 8;
- Fig. 12: einen Schnitt durch die Vorrichtung Figur 11;
- Fig. 13: eine perspektivische Ansicht einer alternativen Ausführungsform der Vorrichtung nach Figur 8;
- Fig. 14: einen Schnitt durch die Vorrichtung nach Figur 13;
- Fig. 15: eine perspektivische Ansicht einer alternative Ausführungsform einer Vorrichtung nach der Erfindung;
- Fig. 16: einen Schnitt durch eine alternative Ausführungsform der Vorrichtung nach Figur 15;
- Fig. 17: eine Seitenansicht einer alternativen Ausführungsform einer Vorrichtung nach der Erfindung;
- Fig. 18: eine Seitenansicht einer alternativen Ausführungsform der Vorrichtung nach Figur 17;
- Fig. 19: eine Seitenansicht einer alternativen Ausführungsform einer Vorrichtung nach der Erfindung;
- Fig. 20: eine perspektivische Ansicht einer alternativen Ausführungsform einer Vorrichtung nach der Erfindung;
- Fig. 21: eine Seitenansicht der Vorrichtung nach Figur 20;
- Fig. 22: einen Schnitt durch die Vorrichtung nach Figur 20;
- Fig. 23: eine perspektivische Ansicht einer alternativen Ausführungsform einer Vorrichtung nach der Erfindung;
- Fig. 24: eine Seitenansicht der Vorrichtung nach Fig. 23;
- Fig. 25: einen Schnitt durch die Vorrichtung nach Fig. 23 entlang der Linie XXIV-XXIV in Fig. 24;
- Fig. 26a: eine perspektivische Ansicht einer alternativen Ausführungsform einer Vorrichtung nach der Erfindung;
- Fig. 26b: eine perspektivische Ansicht einer alternativen Ausführungsform der Vorrichtung nach Figur 26a;
- Fig. 27: einen Schnitt durch die Vorrichtung nach Fig. 26a;
- Fig. 28: eine perspektivische Ansicht einer alternativen Ausführungsform einer Vorrichtung nach der Erfindung;
- Fig. 29: einen Schnitt durch die Vorrichtung nach Fig. 28;
- Fig. 30: eine perspektivische Ansicht einer alternativen Ausführungsform einer Vorrichtung nach der Erfindung;
- Fig. 31: einen Schnitt durch die Vorrichtung nach Fig. 30;
- Fig. 32: eine perspektivische Ansicht einer alternativen Ausführungsform der Vorrichtung nach Fig. 30;
- Fig. 33: einen Schnitt durch die Vorrichtung nach Fig. 32;
- Fig. 34: eine perspektivische Ansicht einer alternativen Ausführungsform der Vorrichtung nach Figur 30;
- Fig. 35: einen Schnitt durch die Vorrichtung nach Fig. 34;

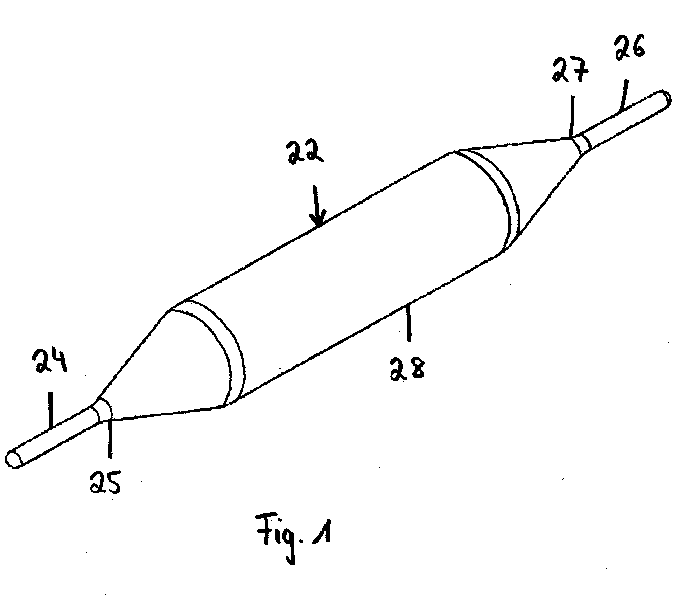

In Figur 1 ist eine Kapsel 22 dargestellt, die einen fließfähigen Stoff, insbesondere einen medizinischen Stoff, einen pharmazeutischen Stoff, ein Nahrungsergänzungsmittel oder ein Kosmetikum enthält. Die Kapsel 22 hat einen Hauptkörper 28, der sich an beiden Seiten jeweils in eine erste Spitze 24 und eine zweite Spitze 26 verjüngt. Zwischen dem Hauptkörper 28 und den beiden Spitzen 24 und 26 befindet sich jeweils eine Sollbruchstelle 25 bzw. 27, an der die Spitzen 24 bzw. 26 von dem Körper abbrechbar sind.

Durch Anpassung eines Öffnungsdurchmessers der Kapsel 22, der nach dem Abbrechen der Spitzen 24 bzw. 26 jeweils an der Sollbruchstelle 25 bzw. 27 entsteht, und durch Anpassung einer Viskosität des fließfähigen Stoffes wird eine Fließ- bzw. Austrittgeschwindigkeit des fließfähigen Stoffes aus der Kapsel 22 gesteuert. Wird eine Spitze 24 bzw. 26 der Kapsel 22 entfernt, tritt der fließfähige Stoff tröpfchenweise aus der Kapsel 22 aus. Bei Entfernung beider Spitzen 24, 26 erhöht sich die Fließgeschwindigkeit.

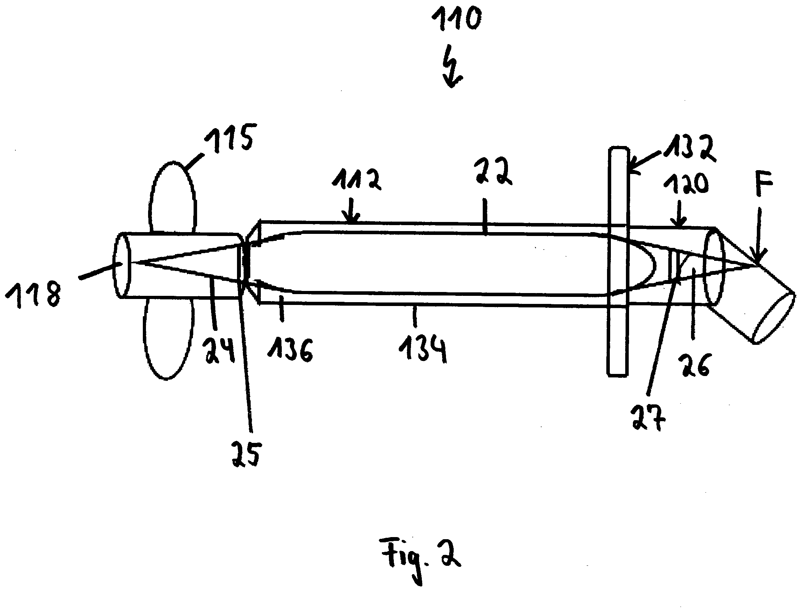

In Figur 2 ist eine Vorrichtung 110 zum Applizieren des fließfähigen Stoffes in einer Betätigungsstellung einer Aktivierungseinrichtung 120 dargestellt. Die Vorrichtung 110 hat eine Körper bzw. eine Applikatorhülse 112, wobei die Applikatorhülse 112 mit einem Wandbereich 134 einen Aufnahmeraum 136 für die Kapsel 22 bildet und an einem Ende der Applikatorhülse 112 die Aktivierungseinrichtung 120 angeordnet ist. Die Aktivierungseinrichtung 120 ist aus einem flexiblen Material und derart an der Applikatorhülse 112 angebracht, dass sich die zweite Spitze 26 der Kapsel 22 in dieser befindet. Somit kann durch ein Betätigen bzw. ein Umbiegen der Aktivierungseinrichtung 120 die zweite Spitze 26 der Kapsel 22 an der Sollbruchstelle 27 abgebrochen werden.

In einem der Aktivierungseinrichtung 120 abgewandten Bereich verjüngt sich die Applikatorhülse 112 zu einem Endabschnitt 114 mit einer Austragöffnung 118 und einem Greifelemente 115. Der Endabschnitt 114 nimmt die erste Spitze 24 der Kapsel 22 auf, so dass ein Nutzer die erste Spitze 24 der Kapsel 22 abbrechen kann, ohne direkt mit der ersten Spitze 24 in Kontakt zu kommen.

An einem der Aktivierungseinrichtung 120 zugewandten Endbereich der Applikatorhülse 112 hat die Vorrichtung 110 eine Greifhilfe 132, die eine gute Bedienbarkeit der Vorrichtung ermöglicht.

Die Aktivierungseinrichtung 120 kann eine Vakuum-Kammer sein, wodurch in der Aktivierungseinrichtung 120 ein Unterdruck herrscht. Dies ermöglicht es, dass nach einem Entfernen der beiden Spitzen 24 und 26 der in der Kapsel 22 beinhaltete fließfähige Stoff definiert auf einen gewünschten Bereich applizierbar ist. Die Vorrichtung 110 wirkt somit wie eine herkömmliche Arzneimittelpipette und ein unkontrolliertes Herausfließen des fließfähigen Stoffes wird verhindert.

Wird die Aktivierungseinrichtung 120 ohne besondere Vorkehrungen bzgl. des in dieser herrschenden Drucks an der Applikatorhülse 112 fixiert, fließt bzw. spritzt der in der Kapsel 22 enthaltene Stoff nach Entfernen der beiden Spitzen 24 und 26 mit vergleichsweise hoher Geschwindigkeit aus der Kapsel 22.

Die Aktivierungseinrichtung 120 kann mit einem Kennzeichen bzw. einem Hinweis versehen sein, das eine korrekte Betätigung der Aktivierungseinrichtung 120 anzeigt.

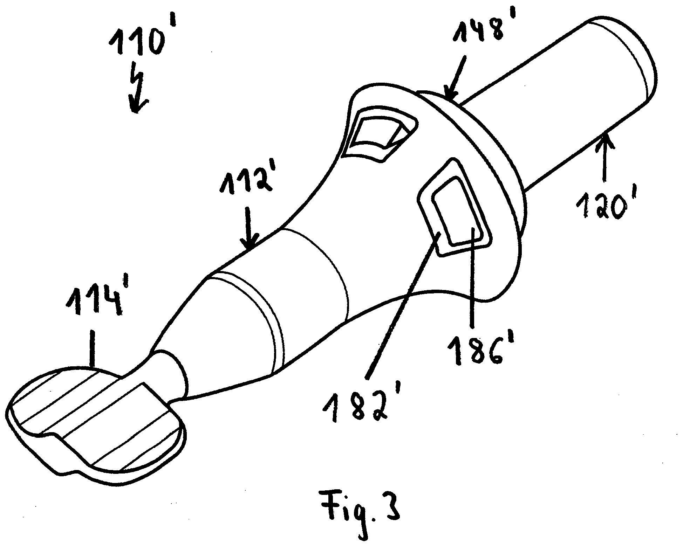

Figur 3 zeigt eine alternative Ausführungsform einer Vorrichtung 110' im Sinne der in Figur 2 dargestellten Vorrichtung 110 nach der Erfindung, wobei Merkmale, die im Folgenden nicht näher erläutert sind, denen der Vorrichtung 110 aus Figur 2 entsprechen.

Die Vorrichtung 110' hat eine Applikatorhülse 112', die in ihrer Form der Form der Kapsel 22 ähnelt, mit der Ausnahme, dass sich die Wanddicke in einem einer Aktivierungseinrichtung 120' zugewandten Bereich vergrößert. Durch die Vergrößerung der Wanddicke bildet die Applikatorhülse 112' eine Greifhilfe 132` mit einer eine Ausnehmungen 182' aufweisenden Deckelfläche 172' aus.

Zudem umfasst die Vorrichtung 110'ein Fixierelement 148' mit Zähnen 186`, die mit den Ausnehmungen 182' der Deckelfläche 172' korrespondieren und bei einem Zusammenbau bzw. einer Montage der Vorrichtung 110'verrasten. Somit sind die Zähne 186` in den Ausnehmungen 182' und die Aktivierungseinrichtung 120' an der Applikatorhülse 112' fixiert.

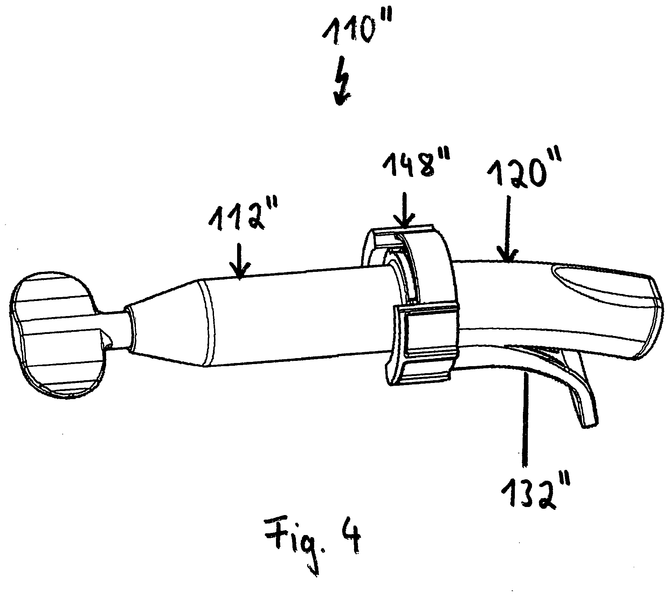

In Figur 4 ist eine zweite alternative Ausführungsform einer Vorrichtung 110" im Sinne der in Figur 2 dargestellten Vorrichtung 110 nach der Erfindung dargestellt, wobei sich diese von den zuvor dargestellten Ausführungsformen dadurch unterscheidet, dass ein Fixierelement 148" mittels eines Gewindes an einer Applikatorhülse 112" gesichert ist und das Fixierelement 148" eine Greifhilfe 132" hat.

Die Greifhilfe 132" ist an dem Fixierelement 148" angebunden und erstreckt sich entlang einer Aktivierungseinrichtung 120", wobei sich der Abstand der Greifhilfe 132" zur Aktivierungseinrichtung 120" ausgehend von dem Fixierelement 148" vergrößert.

Die Greifhilfe 132" ist derart geformt, dass sie einem Nutzer angenehm in der Hand liegt. Hierzu sind Kanten der Greifhilfe 132", die sich auf einer der Nutzerhand zugewandten Seite befinden, abgerundet.

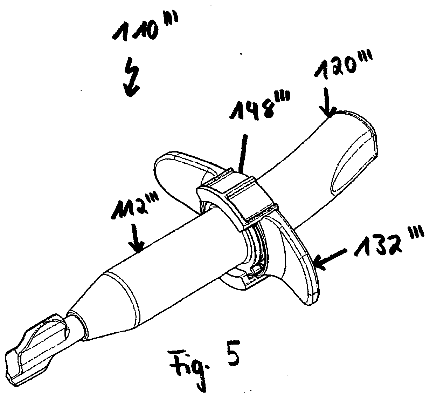

Figur 5 zeigt eine weitere alternative Ausführungsform einer Vorrichtung 110`" im Sinne der in Figur 2 dargestellten Vorrichtung 110 nach der Erfindung, die sich von der in Figur 2 dargestellten Vorrichtung 110 durch die Anbindung einer Aktivierungseinrichtung 120" ` an einer Applikatorhülse 112‴ und durch die Ausgestaltung einer Greifhilfe 132`" unterscheidet. Die anderen Merkmale entsprechen den Merkmalen der Vorrichtung 110.

Die Aktivierungseinrichtung 120‴ hat an einem der Applikatorhülse 112‴ zugewandten Ende einen Flansch 121‴ mittels dem sie durch ein ein Gewinde aufweisendes Fixierelement 148‴ an der Applikatorhülse 112‴ fixiert ist. Das Fixierelement 148‴ umfasst die Greifhilfe 132'".

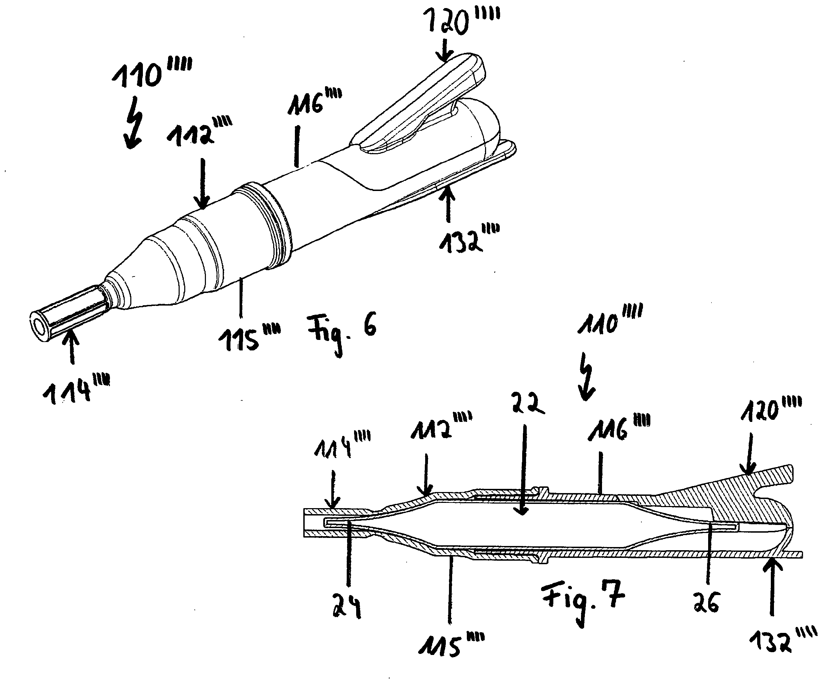

In den Figuren 6 und 7 ist eine weitere alternative Ausführungsform der Vorrichtung 110"" nach Figur 2 dargestellt, wobei im Folgenden nicht näher erläuterte Merkmale den Merkmalen der in Figur 2 dargestellten Vorrichtung 110 entsprechen.

Die Vorrichtung 110ʺʺ umfasst eine Applikatorhülse 112ʺʺ mit einem vorderen Hauptkörper 115"" und einem hinteren Hauptkörper 116"". Der vordere Hauptkörper 115ʺʺ weist an einem der ersten Spitze 24 der Kapsel 22 zugewandten Ende einen Endabschnitt 114ʺʺ auf. Der Endabschnitt 114"" ist abbrechbar, wobei durch ein Abbrechen des Endabschnitts 114ʺʺ auch die erste Spitze 24 der Kapsel 22 abbricht, so dass der in der Kapsel beinhalteten fließfähigen Stoffes applizierbar ist.

Der hintere Hauptkörper 116"" hat an seinem dem zweiten Ende 26 der Kapsel zugewandten Ende eine Aktivierungseinrichtung 120ʺʺ, die nach Art eines Hebels ausgebildet ist. Die Aktivierungseinrichtung 120ʺʺ ist aus einem flexiblen Material, so dass durch Betätigung dieser, d.h. durch Beaufschlagung der Aktivierungseinrichtung 120ʺʺ mit einer in radiale Richtung der Vorrichtung 110ʺʺ wirkenden Kraft, die zweite Spitze 26 der Kapsel 22 abbricht. Zudem ist aufgrund des flexiblen Materials der Aktivierungseinrichtung 120ʺʺ ein definiertes applizieren bzw. pipettieren des fließfähigen Stoffes möglich.

An einer der Aktivierungseinrichtung 120ʺʺ in Umfangrichtung gegenüberliegenden Seite des hinteren Hauptkörpers 116ʺʺ ist eine Greifhilfe 132ʺʺ in Form einer eben ausgestalteten Fläche angeordnet.

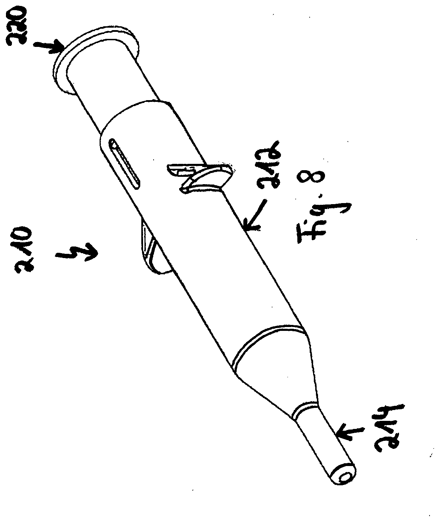

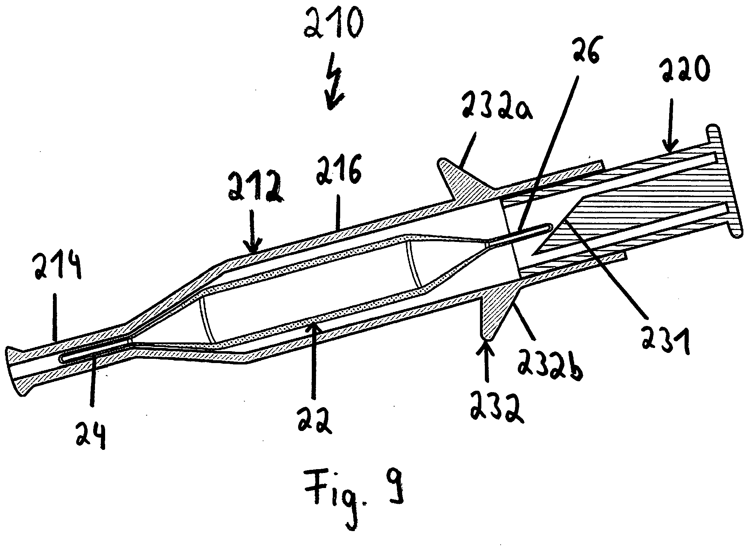

In den Figuren 8 und 9 ist eine alternative Ausführungsform einer Vorrichtung 210 nach der Erfindung dargestellt.

Die Vorrichtung 210 hat eine Applikatorhülse 212, die die Kapsel 22 beinhaltet und sich in einem einer Aktivierungseinrichtung 220 abgewandten Bereich von einem zylindrischen Hauptkörper 216 in einen Endabschnitt 214 verjüngt, und eine Aktivierungseinrichtung 220.

Wie Figur 9 zeigt, ist die Aktivierungseinrichtung 220 ein Kolben, der eine lineare und asymmetrisch auf die Spitze 26 der Kapsel 22 wirkende Abschrägung 231 hat. Mittels der Abschrägung 231 kann die zweite Spitze 26 der Kapsel 22 an der Sollbruchstelle 27 auf optimierte Weise abgebrochen werden.

Die Applikatorhülse 212 hat eine Greifhilfe 232 aus zwei sich gegenüberliegenden hervorstehenden Elementen 232a, 232b, die es einem Nutzer ermöglicht, mit zwei Fingern, insbesondere einem Ringfinger und einem Zeigefinger, die Vorrichtung 210 derart zu greifen, dass mit der Handinnenfläche oder dem Daumen die Aktivierungseinrichtung 220 aktiviert werden kann, wodurch die Aktivierungseinrichtung 220 in axialer Richtung in die Applikatorhülse 212 verschoben und die zweite Spitze 26 der Kapsel 22 indirekt abgebrochen wird.

An der Applikatorhülse 212 ist ein sich in axialer Richtung erstreckender Schlitz 240 ausgebildet. Die Aktivierungseinrichtung 220 hat einen mit dem Schlitz 240 der Applikatorhülse 212 korrespondierenden und in diesem angeordneten Zapfen 238. Somit kann durch den Schlitz 240 ein Bewegungsweg des Zapfens 238 und folglich ein Bewegungsspielraum der Aktivierungseinrichtung 220 relativ zur Applikatorhülse 212 definiert bzw. begrenzt werden.

Mittels des Zapfens 238 und des Schlitzes 240 kann demzufolge sichergestellt werden, dass die Aktivierungseinrichtung 220 bei einer Betätigung nur so weit in Richtung der Kapsel 22, das heißt in axialer Richtung, verschoben wird, dass die zweite Spitze 26 abbricht, eine Beschädigung des Hauptkörpers 28 der Kapsel 22 jedoch ausgeschlossen ist.

Durch eine derartige Betätigung der Aktivierungseinrichtung 220 und ein Abbrechen des Endabschnitts 214 ist der in der Kapsel 22 beinhaltete fließfähige Stoff applizierbar.

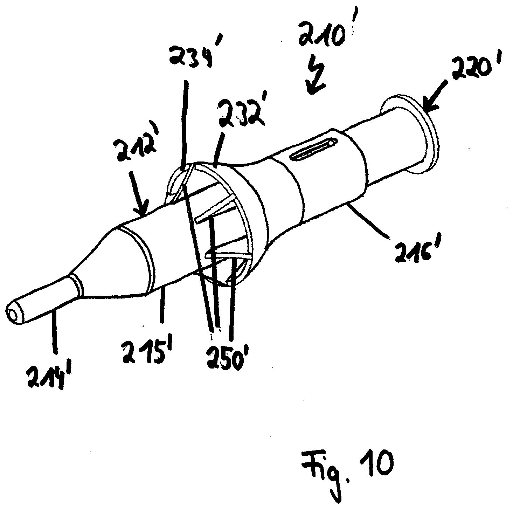

Figur 10 zeigt eine alternative Ausführungsform einer Vorrichtung 210`, insbesondere einer Ausgestaltung einer Applikatorhülse 212' und einer Greifhilfe 232` der in den Figuren 8 und 9 dargestellten Vorrichtung 210. Alle anderen Merkmale der Vorrichtung 210` entsprechen der in den Figuren 8 und 9 gezeigten Vorrichtung 210.

Die Applikatorhülse 212' hat einen vorderen Hauptkörperabschnitt 215` und einen hinteren Hauptkörperabschnitt 216`. Der vordere Hauptkörperabschnitt 215` ist zylindrisch und verjüngt sich in einen Endabschnitt 214`. Der hintere Hauptkörperabschnitt 216` ist in einem der Aktivierungseinrichtung 220' zugewandten Bereich ebenfalls zylindrisch. In einem dem vorderen Hauptkörperabschnitt 215' zugewandten Bereich vergrößert sich der Durchmesser des zylinderförmigen hinteren Hauptkörperabschnitt 216`, wobei die Dicke eines die Applikatorhülse 212' bildenden Wandbereichs 234` konstant ist. Der hintere Hauptkörperabschnitt 216` endet ca. auf Höhe der Mitte der Kapsel 22. Der vordere Hauptkörperabschnitt 215' erstreckt sich in Richtung des hinteren Hauptkörperabschnitts 216` bzw. in Richtung der Aktivierungseinrichtung 220' bis er mit dem hinteren Hauptkörperabschnitt 216` kontaktiert. Somit ist in einem mittleren Bereich der Applikatorhülse 212' der Durchmesser des hinteren Hauptkörperabschnitts 216` größer als der des vorderen Hauptkörperabschnitts 215`, wobei die beiden Hauptkörperabschnitte 215" und 216' mittels Rippen 250' verbunden sind.

Die aus der Vergrößerung des Durchmessers des hinteren Hauptkörperabschnitts 216` resultierende Erhebung der Applikatorhülse 212' bildet die Greifhilfe 232', wobei die Rippen 250' die Greifhilfe 232` stabilisieren.

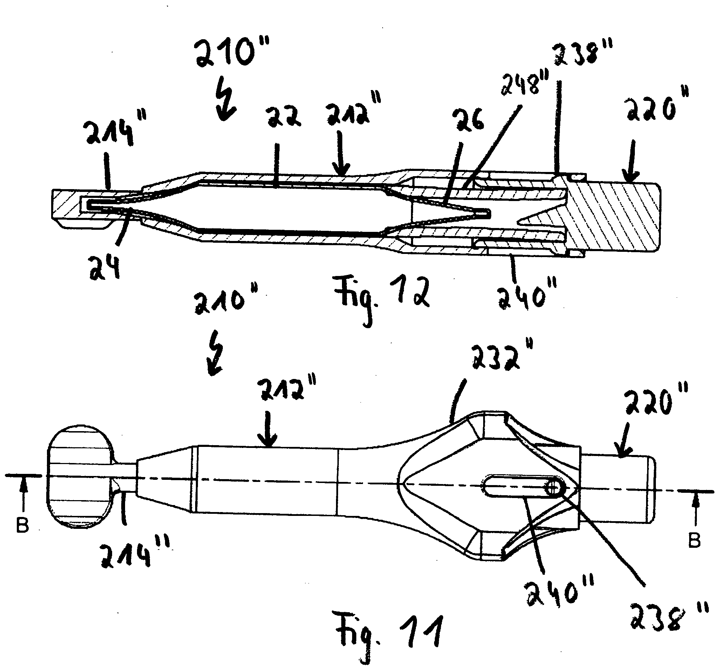

Die in den Figuren 11 und 12 dargestellte Ausführungsform einer Vorrichtung 210" unterscheidet sich von der in den Figuren 8 und 9 gezeigten Vorrichtung 210 durch eine Form einer Applikatorhülse 212" und einer Greifhilfe 232 " und dadurch, dass zwischen der Applikatorhülse 212" und einer Aktivierungseinrichtung 220" ein Rückstellelement 248" angeordnet ist, das die Aktivierungseinrichtung 220" nach einem axialen Verschieben in Richtung eines Endabschnitts 214" in deren Ausgangsstellung zurückführt.

Eine Integration eines derartiges Rückstellelements 248" ist auch bei den anderen Ausführungsformen denkbar.

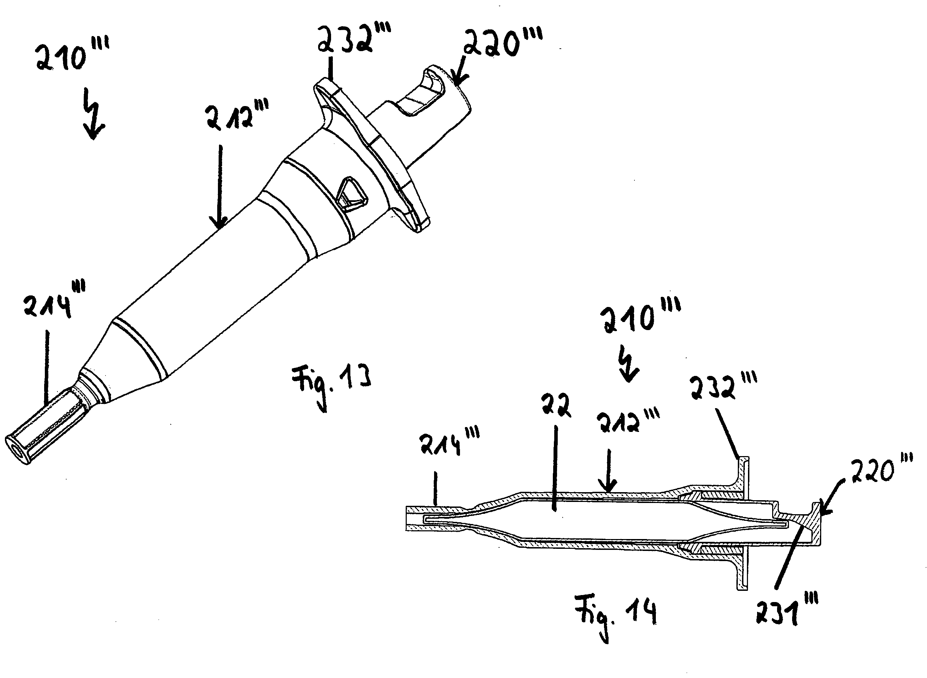

In den Figuren 13 und 14 ist eine alternative Ausführungsform einer Vorrichtung 210`" der in den Figuren 8 und 9 gezeigten Vorrichtung 210 dargestellt, wobei im Folgenden lediglich Merkmale der Vorrichtung 210‴ ` näher erläutert werden, die sich von der Vorrichtung 210 unterscheiden.

Die Vorrichtung 210‴ umfasst eine Greifhilfe 232‴, die am der zweiten Spitze 26 der Kapsel 22 zugewandten Ende angeordnet ist, und eine Aktivierungseinrichtung 220"'. Die Aktivierungseinrichtung 220‴ ist ein Kolben, der eine lineare und asymmetrisch auf die Spitze 26 der Kapsel 22 wirkende Abschrägung 231‴ hat. Mittels der Abschrägung 231‴ kann die zweite Spitze 26 der Kapsel 22 an der Sollbruchstelle 27 auf optimierte Weise abgebrochen werden.

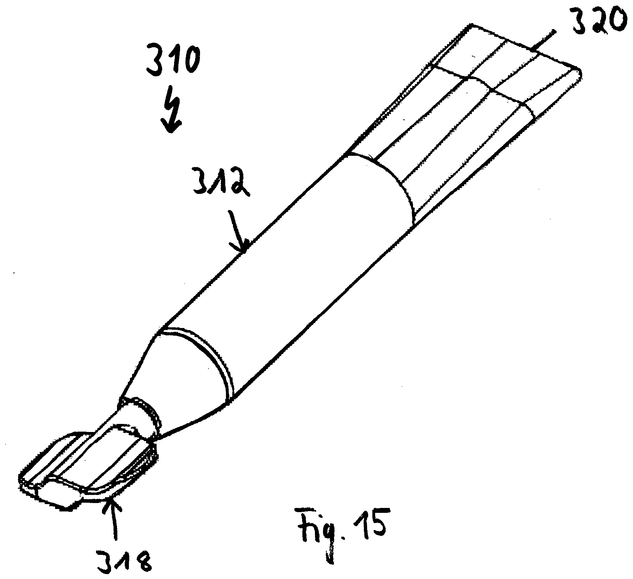

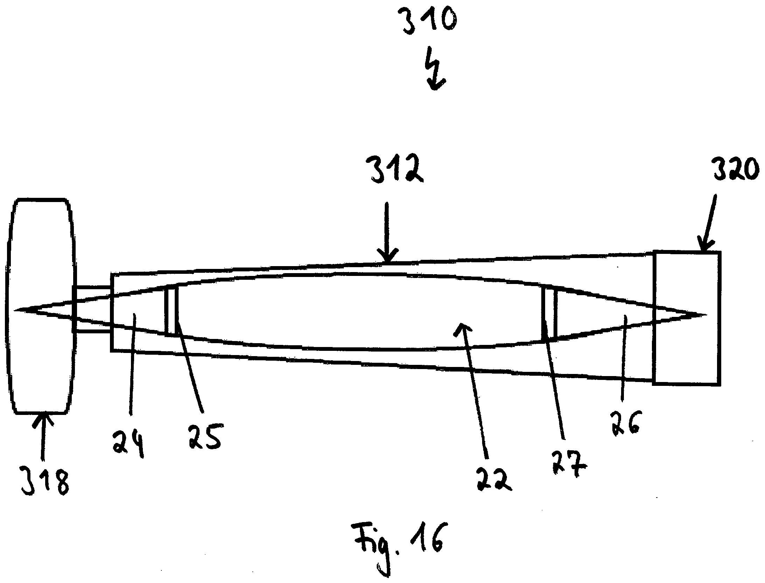

Eine weitere alternative Ausführungsform einer Vorrichtung 310 nach der Erfindung ist in den Figuren 15 und 16 dargestellt. Die Vorrichtung 310 umfasst eine steife Tube 312, welche die Kapsel 22 beinhaltet. Die Tube 312 hat an einem Ende einen Verschluss 318 und an dem anderen Ende eine als eine Aktivierungseinrichtung dienende Schweißnaht 320. Der Verschluss 318 ist ein Abknickverschluss und kann Greifelemente aufweisen.

Vor einem Zusammenbau der Vorrichtung 310 weist die Tube 312 auf einer dem Verschluss 318 abgewandten Seite eine Öffnung auf, durch welche die Kapsel 22 in die Tube 312 eingeführt wird, so dass sich der Kapselkörper 28 und die erste Spitze 24 der Kapsel 22 in der Tube 312 und ein Endabschnitt der zweiten Spitze 26 oder die zweite Spitze 26 in der Öffnung der Tube 312 befinden. Die Öffnung der Tube 312 wird anschließend verschlossen, insbesondere verschweißt, so dass die zweite Spitze 26 in der Schweißnaht 320 bzw. einer Schweißfläche integriert ist.

Die Schweißnaht 320 ist flexibel bzw. deformierbar. Somit kann durch ein Abknicken der Schweißnaht 320 die zweite Spitze 26 der Kapsel 22 abgebrochen werden. Durch ein anschließendes Entfernen der ersten Spitze 24 der Kapsel 22 ist der fließfähige Stoff somit applizierbar.

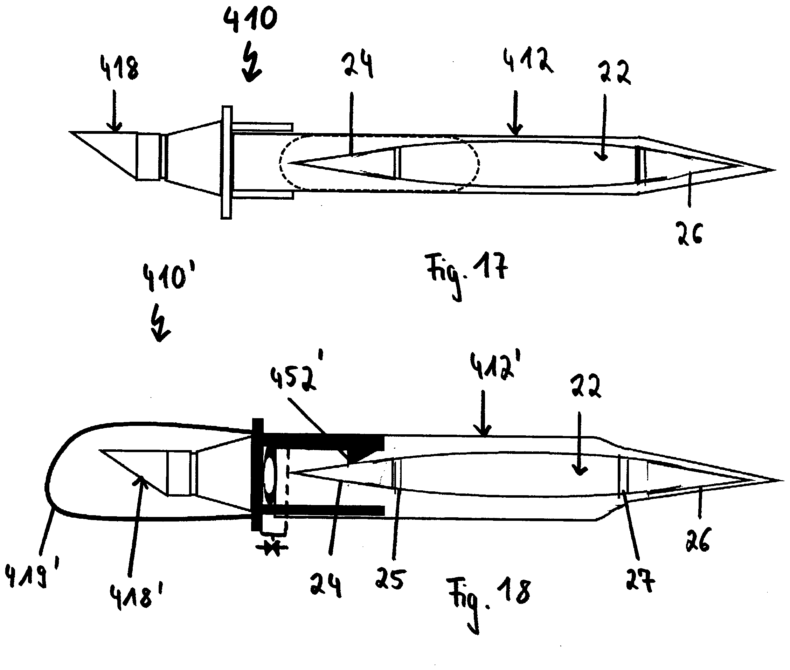

In der Figur 17 ist eine alternative Ausführungsform einer Vorrichtung 410 nach der Erfindung dargestellt.

Die Vorrichtung 410 umfasst eine die Kapsel 22 beinhaltende Applikatorhülse 412 und einen Applikator 418. Die Applikatorhülse 412 ist in einem dem Applikator 418 zugewandten Bereich und in einem dem Applikator 418 abgewandten Endabschnitt deformierbar, wodurch die beiden Spitzen 24 und 26 der Kapsel abbrechbar sind. Die Applikatorhülse 412 ist somit selbst eine Aktivierungseinrichtung.

Der Applikator 418 besteht aus einem saugfähigen Material. Das saugfähige Material nimmt nach dem Abbrechen der beiden Spitzen 24 und 26 den fließfähigen Stoff auf der der Applikatorhülse 412 zugewandten Seite auf und gibt diesen anschließend an der gegenüberliegenden Seite definiert ab.

Die in Figur 18 dargestellte Ausführungsform einer Vorrichtung 410' unterscheidet sich von der in Figur 17 gezeigten Vorrichtung 410 dadurch, dass ein Abbrechen der ersten Spitze 24 der Kapsel 22 durch ein Stauchen einer Applikatorhülse 412' in einem dem Applikator 418' zugewandten Bereich erfolgt. Hierzu hat die Vorrichtung 410`einen asymmetrischen Kragen 452`, der bei einem axialen Verschieben der Kapsel 22 in Richtung dem Applikator 418' die erste Spitze 24 abbricht. Die Vorrichtung 410 ` umfasst des Weiteren eine Schutzkappe 419`.

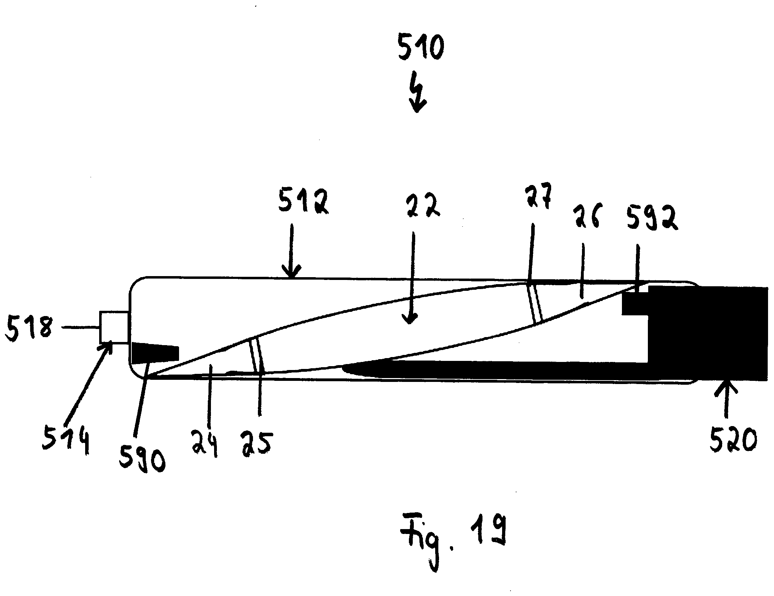

Eine weitere alternative Ausführungsform einer Vorrichtung 510 nach der Erfindung ist in Figur 19 gezeigt.

Die Vorrichtung 510 hat eine die Kapsel 22 beinhaltende Applikatorhülse 512. Die Position der Kapsel 22 in der Applikatorhülse 512 ist mittels eines Vorsprungs 590 auf der Innenseite der Applikatorhülse 512 sowie eines exzentrisch angeordneten Vorsprungs 592 einer als Kolben ausgestaltete Aktivierungseinrichtung 520 definiert. Durch ein axiales Verschieben der Aktivierungseinrichtung 520 in Richtung eines Endabschnitts 514 werden die erste Spitze 24 der Kapsel 22 durch den Vorsprung 590 und die zweite Spitze 26 der Kapsel 22 durch den Vorsprung 592 abgebrochen. Der Endabschnitt 514 ist an einer der Aktivierungseinrichtung 520 abgewandten Seite angeordnet und hat eine Öffnung 518.

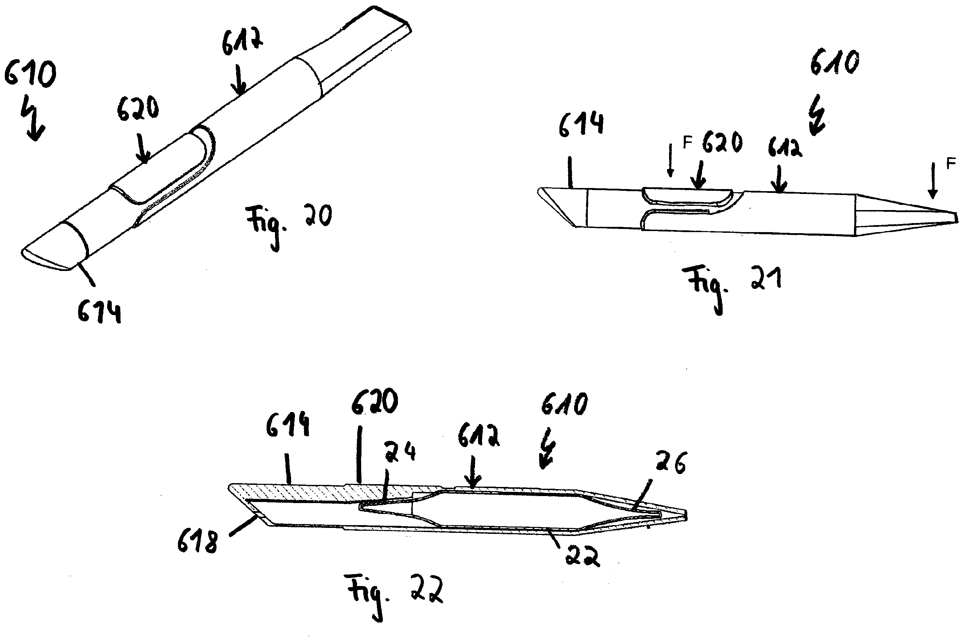

In den Figuren 20 bis 22 ist eine weitere alternative Ausführungsform einer Vorrichtung 610 nach der Erfindung dargestellt.

Die Vorrichtung 610 hat eine Applikatorhülse 612 mit einem eine Austrageöffnung 618 aufweisenden Endabschnitt 614 und eine Aktivierungseinrichtung 620. Die Aktivierungseinrichtung 620 ist auf Höhe der ersten Spitze 24 der Kapsel 22 angeordnet und durch ein Bewegen in radiale Richtung betätigbar, wobei durch ein Betätigen der Aktivierungseinrichtung 620 die erste Spitze 24 der Kapsel 22 abbricht. Auf der dem Endabschnitt 614 abgewandten Seite ist die Applikatorhülse 612 aus einem elastischen Material. Somit kann durch ein Umbiegen des elastischen Teils der Applikatorhülse 612 die zweite Spitze 26 der Kapsel 22 abgebrochen werden. Mittels Betätigung der Aktivierungseinrichtung 620 wird anschließend eine definierte Menge des in der Kapsel 22 beinhalteten fließfähigen Stoffes durch die Austrageöffnung 618 appliziert.

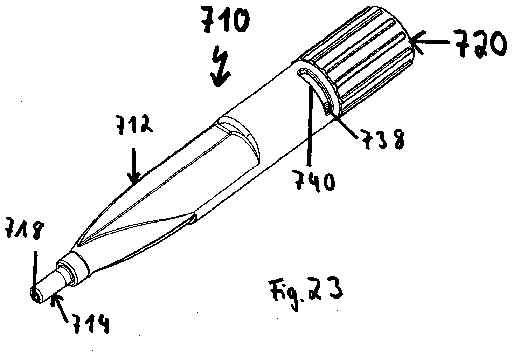

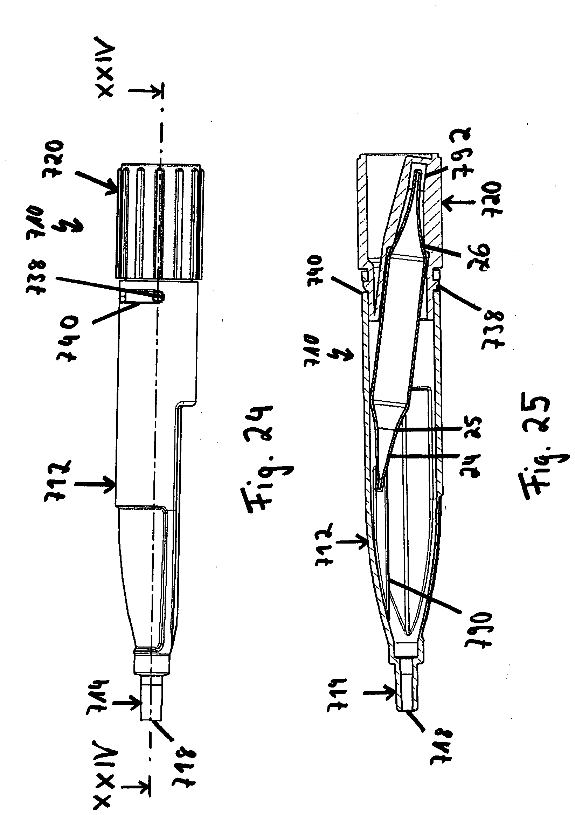

Eine weitere Ausführungsform einer Vorrichtung 710 nach der Erfindung ist in den Figuren 23 bis 25 dargestellt. Die Vorrichtung 710 hat eine Applikatorhülse 712 mit einem Endabschnitt 714 und eine Aktivierungseinrichtung 720, die relativ zur Applikatorhülse 712 um die Längsachse der Vorrichtung 710 drehbar ist. Der Endabschnitt 714 hat eine Austrageöffnung 718. Die Kapsel 22 ist in der Vorrichtung 710, insbesondere der Applikatorhülse 712 und der Aktivierungseinrichtung 720 angeordnet. Eine erste Spitze 24 der Kapsel 22 ist mittels eines Positionierelements 790 der Applikatorhülse 712 positioniert. Die Aktivierungseinrichtung 720 hat ebenfalls ein Positionierelement 792, das die zweite Spitze 26 der Kapsel 22 sichert.

Durch die Sicherung der Kapsel 22 mittels der Positionierelemente 790 und 792 brechen die erste und die zweite Spitze 24, 26 bei einer Rotation der Aktivierungseinrichtung 720 ab, so dass der in der Kapsel 22 enthaltende fließfähige Stoff durch die Austrageöffnung applizierbar ist. Der Rotationsspielraum der Aktivierungseinrichtung 720 relativ zur Applikatorhülse 712 um die Vorrichtungslängsachse ist durch einen in der Applikatorhülse 712 vorgesehener Schlitz 740 und einen an der Aktivierungseinrichtung 720 ausgebildeten Zapfen 738 begrenzt.

Alternativ kann die Vorrichtung 710 auch ohne Zapfen 738 und Schlitz 740 ausgestaltet sein, so dass eine Rotation der Aktivierungseinrichtung 720 relativ zur Applikatorhülse 712 unbegrenzt möglich ist.

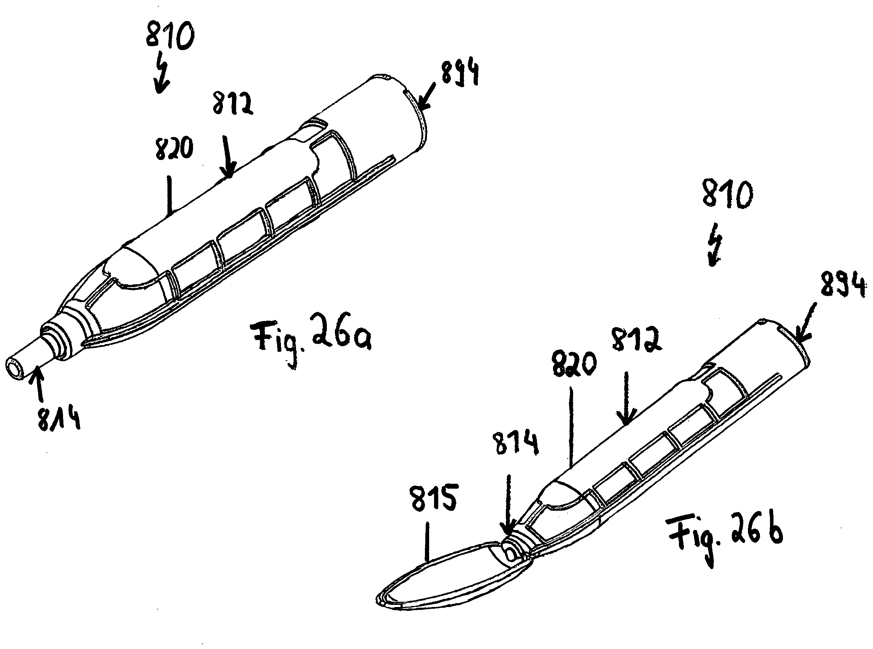

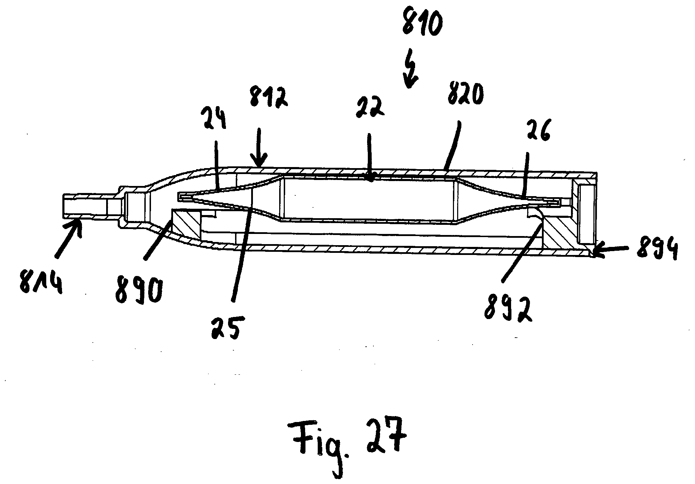

In den Figuren 26a, 26b und 27 ist eine weitere Ausführungsform einer Vorrichtung 810 nach der Erfindung dargestellt. Die Vorrichtung 810 hat eine Applikatorhülse 812, die in einem in radialer Richtung oberen und in axialer Richtung mittleren Abschnitt eine Aktivierungseinrichtung 820 in Form eines elastischen Bereichs umfasst.

Die Applikatorhülse 812 hat an ihrem vorderen Ende einen Endabschnitt 814. Der Endabschnitt 814 kann ein in Figur 26b dargestelltes löffelförmiges Aufnahmegefäß 815 umfassen. An ihrem hinteren Ende hat die Applikatorhülse 812 eine Öffnung, in der sich eine Positioniereinrichtung 894 befindet. Die Positioniereinrichtung 894 erstreckt sich in Richtung des Endabschnitts 814 und hat zwei Positionierelemente 890 und 892, mittels welcher die Kapsel 22 über ihre beiden Spitzen 24 und 26 in der Vorrichtung 810 positionsgesichert ist.

Durch Betätigung der Aktivierungseinrichtung 820, das heißt durch Ausüben eines Drucks auf den elastischen Bereich der Applikatorhülse 812 in radialer Richtung, erfährt der Hauptkörper 28 der Kapsel eine Belastung in radialer Richtung, so dass die beiden positionsgesicherten Spitzen 24 und 26 der Kapsel 22 abbrechen, der fließfähige Stoff aus der Kapsel fließt und mittels des Endabschnitts 814 appliziert werden kann.

Die Applikatorhülse 812 der Vorrichtung 810 kann auch zweiteilig mit einem steifen Grundkörper und einem flexiblen Deckelelement ausgestaltet sein. Das flexible Deckelelement ist beispielsweise mittels einer Clip-Verbindung an dem Grundkörper fixiert und bildet die Aktivierungseinrichtung.

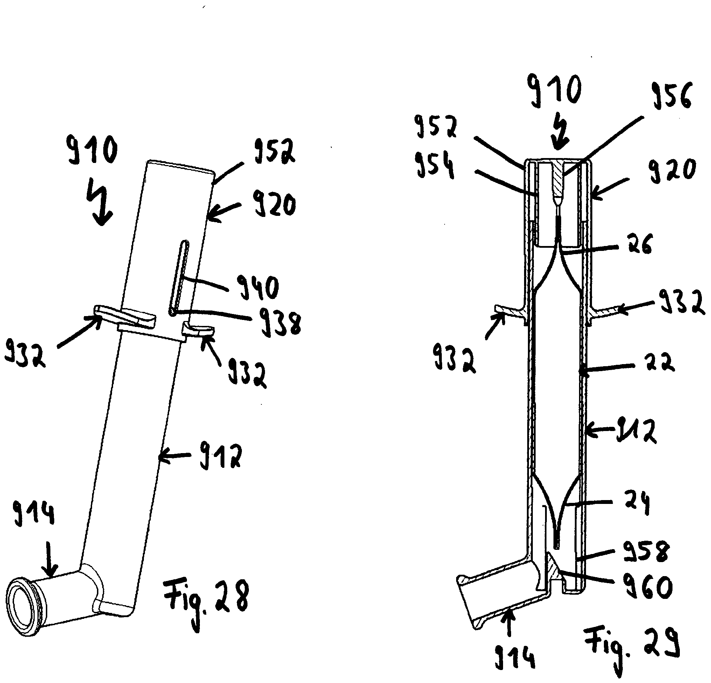

Eine weitere alternative Ausführungsform einer Vorrichtung 910 nach der Erfindung ist in den Figuren 28 und 29 dargestellt. Die Vorrichtung 910 hat eine Applikatorhülse 912 mit einer Greifhilfe 932, in der die Kapsel 22 angeordnet ist. An einem Ende hat die Applikatorhülse 912 ein Positionierelement 958 mit einer Abschrägung 960 und einen rohrförmigen Endabschnitt 914, der sich in eine Richtung ungleich der Richtung des axialen Verlaufs der Applikatorhülse 912 erstreckt.

An dem anderen Ende der Applikatorhülse 912 ist eine Aktivierungseinrichtung 920 angeordnet, die eine zylindrische Außenwand 952, eine zylindrische Innenwand 954, einen Schlitz 940 und ein Aktivierungselement 956 hat. Die Aktivierungseinrichtung 920 ist derart an der Applikatorhülse 912 montiert, dass sich die Wand der Applikatorhülse 912 zwischen der Außenwand 952 und der Innenwand 954 der Aktivierungseinrichtung 920 befindet. Ein an der Applikatohülse 912 ausgebildeter Zapfen 938, der mit dem Schlitz 940 der Aktivierungseinrichtung 956 korrespondiert, ist in dem Schlitz 940 der Aktivierungseinrichtung 920 angeordnet.

Das Aktivierungselement 956 hat an einem der Kapsel 22 zugewandten Ende eine Abschrägung. Durch Betätigung der Aktivierungseinrichtung 920, das heißt durch Verschieben der Aktivierungseinrichtung 920 in Richtung der Kapsel 22 relativ zur Applikatorhülse 912 kann somit die zweite Spitze 26 der Kapsel 22 mittels des Aktivierungselements 956 und die erste Spitze 24 der Kapsel 22 mittels der Abschrägung 960 des Positionierelements 958 definiert abgebrochen werden.

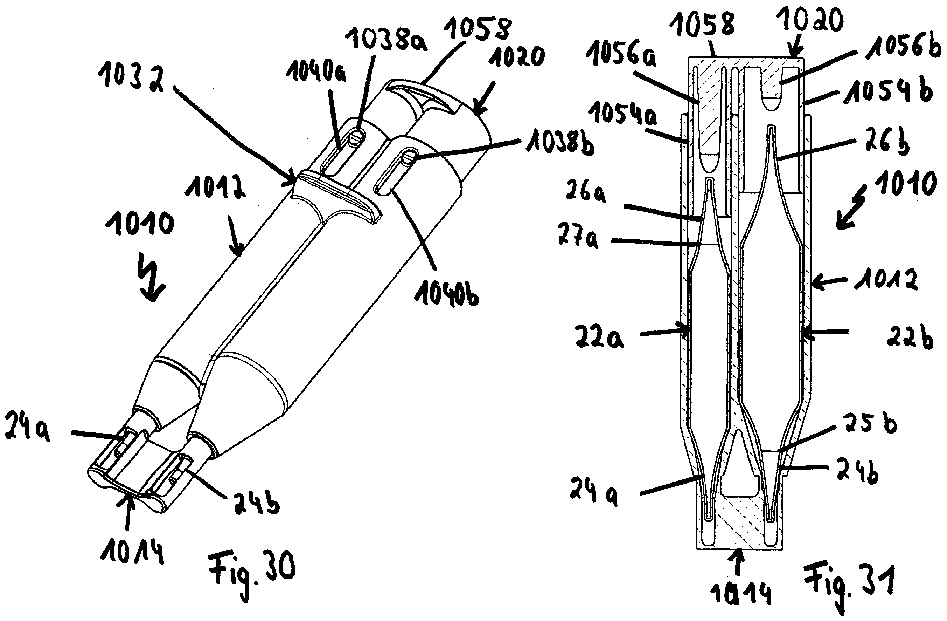

Die Figuren 30 und 31 zeigen eine weitere alternative Ausführungsform einer Vorrichtung 1010 nach der Erfindung.

Die Vorrichtung 1010 umfasst eine Applikatorhülse 1012 bestehend aus zwei zylinderförmigen, parallel verlaufenden und miteinander verbundenen Teilhülsen, die jeweils eine Kapsel 22a bzw. 22b aufnehmen. Die Applikatorhülse 1012 hat eine Greifhilfe 1032. An einem Ende der Applikatorhülse 1012 ist ein Endabschnitt 1014 ausgebildet, der die beiden ersten Spitzen 24a und 24b der Kapseln 22a bzw. 22b beinhaltet. An dem anderen Ende der Applikatorhülse 1012 ist eine Aktivierungseinrichtung 1020 mit zwei zylinderförmigen Teilhülsen 1054a und 1054b, die in einem der Applikatorhülse abgewandten Ende mittels eines Deckelelements 1058 verbunden sind, angeordnet. Der Radius der beiden Teilhülsen 1054a und 1054b ist jeweils kleiner als der Radius, der entsprechenden Teilhülse der Applikatorhülse 1012, so dass die Aktivierungseinrichtung 1020 in der Applikatorhülse 1012 einbringbar bzw. montierbar ist. Die Aktivierungseinrichtung 1020 hat an dem Deckelement 1058 zwei Aktivierungselemente 1056a und 1056b, wobei sich das eine Aktivierungselement 1056a in der ersten Teilhülse 1054a und das andere Aktivierungselement 1056b in der zweiten Teilhülse 1054b befindet und sich die beiden Aktivierungselemente 1056a und 1056b in Richtung der Kapseln 22a bzw. 22b erstrecken.

Durch axiales Verschieben der Aktivierungseinrichtung 1020 relativ zur Applikatorhülse 1012 werden die beiden zweiten Spitzen 26a und 26b der Kapseln 22a und 22b durch an den Aktivierungselementen 1056a und 1056b ausgebildeter Abschrägungen definiert abgebrochen.

Die ersten Spitzen 24a und 24 b der Kapsel 22a bzw. 22b sind durch Biegen bzw. Abbrechen des Endabschnitts 1014 abbrechbar, so dass nach Betätigung der Aktivierungseinrichtung 1020 und Abbrechen des Endabschnitts 1014 der in den Kapsel 22a und 22b beinhaltete fließfähige Stoff ausbringbar ist.

Ein maximaler Verschiebeweg der Aktivierungseinrichtung 1020 relativ zur Applikatorhülse 1012 ist mittels zweier an der Applikatorhülse 1012 vorgesehener Schlitze 1040a und 1040b und an der Aktivierungseinrichtung 1020 ausgebildeter mit den Schlitzen 1040a und 1040b der Applikatorhülse 1012 korrespondierender Zapfen 1038a und 1038b definiert bzw. begrenzt.

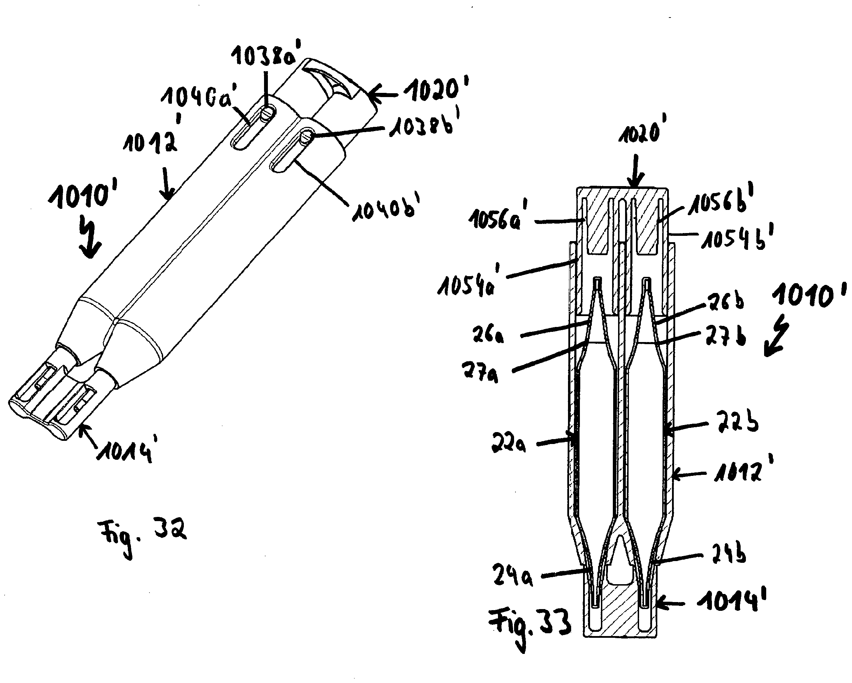

Eine alternative Ausführungsform der Vorrichtung 1010 ist in den Figuren 32 und 33 gezeigt. Die in den Figuren 32 und 33 dargestellte Vorrichtung 1010` unterscheidet sich von der Vorrichtung 1010 lediglich dadurch, dass die beiden in der Vorrichtung 1010` beinhalteten Kapseln gleichgroß sind, so dass sich die beiden Teilhülsen der Applikatorhülse 1012` bzw. die beiden Teilhülsen 1054a` bzw. 1054b` der Aktivierungsvorrichtung 1020' jeweils entsprechen. Des Weiteren hat die Applikatorhülse 1012' keine Greifhilfe. Alle weiteren Merkmale entsprechen denen der Vorrichtung 1010 aus den Figuren 30 und 31.

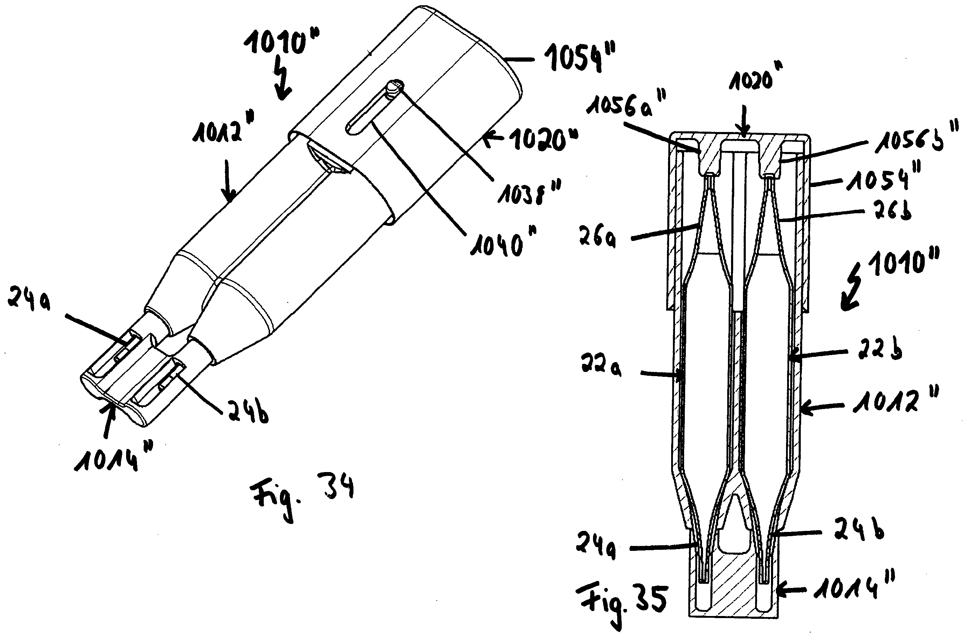

In den Figuren 34 und 35 ist eine alternative Ausführungsform einer Vorrichtung 1010" der in den Figuren 32 und 33 dargestellten Vorrichtung 1020' gezeigt, wobei sich die Vorrichtung 1010" von der Vorrichtung 1010` dadurch unterscheidet, dass die Aktivierungseinrichtung 1020" aus einer Hülse 1054" besteht, die sich außerhalb einer Applikatorhülse 1012" befindet. Die weiteren Merkmale entsprechen denen der Vorrichtung 1010` aus den Figuren 32 und 33.

### Bezugszeichenliste

- 22: Kapsel
- 22a: Kapsel
- 22b: Kapsel
- 24: erste Spitze
- 24a: erste Spitze
- 24b: erste Spitze
- 25: erste Sollbruchstelle
- 26: zweite Spitze
- 26a: zweite Spitze
- 26b: zweite Spitze
- 27: zweite Sollbruchstelle
- 28: Hauptkörper
- 110: Vorrichtung
- 110': Vorrichtung
- 110": Vorrichtung
- 110‴: Vorrichtung
- 110ʺʺ: Vorrichtung
- 112: Applikatorhülse
- 112': Applikatorhülse
- 112": Applikatorhülse
- 112‴: Applikatorhülse
- 112ʺʺ: Applikatorhülse
- 114: Endabschnitt
- 114': Endabschnitt
- 114ʺʺ: Endabschnitt
- 115: Greifelement
- 115ʺʺ: vorderer Hauptkörper
- 116ʺʺ: hinterer Hauptkörper
- 118: Austragöffnung
- 120: Aktivierungseinrichtung
- 120': Aktivierungseinrichtung
- 120ʺ: Aktivierungseinrichtung
- 120‴: Aktivierungseinrichtung
- 120ʺʺ: Aktivierungseinrichtung
- 121‴: Flansch
- 132: Greifhilfe
- 132': Greifhilfe
- 132": Greifhilfe
- 132‴: Greifhilfe
- 132ʺʺ: Greifhilfe
- 134: Wandbereich
- 148': Fixierelement
- 148": Fixierelement
- 148‴: Fixierelement
- 172': Deckelfläche
- 182': Ausnehmung
- 186`: Zähne
- 210: Vorrichtung
- 210': Vorrichtung
- 210": Vorrichtung
- 210‴: Vorrichtung
- 212: Applikatorhülse
- 212': Applikatorhülse
- 212": Applikatorhülse
- 212 ` ` `: Applikatorhülse
- 214: Endabschnitt
- 214`: Endabschnitt
- 214": Endabschnitt
- 214‴: Endabschnitt
- 215': vorderer Hauptkörper
- 216`: hinterer Hauptkörper
- 216: Hauptkörper
- 220: Aktivierungseinrichtung
- 220': Aktivierungseinrichtung
- 220": Aktivierungseinrichtung
- 220‴ `: Aktivierungseinrichtung
- 231: Abschrägung
- 231‴: Abschrägung
- 232: Greifhilfe
- 232a: Element
- 232b: Element
- 232`: Greifhilfe
- 232": Greifhilfe
- 232‴: Greifhilfe
- 234`: Wandbereich
- 238: Zapfen
- 240: Schlitz
- 248": Rückstellelement
- 250': Rippen
- 310: Vorrichtung
- 312: Tube
- 318: Verschluss
- 320: Schweißnaht
- 410: Vorrichtung
- 410': Vorrichtung
- 412: Applikatorhülse
- 412': Applikatohülse
- 418: Applikator
- 418': Applikator
- 419`: Schutzkappe
- 452`: Kragen
- 510: Vorrichtung
- 512: Applikatorhülse
- 514: Endabschnitt
- 518: Austrageöffnung
- 520: Aktivierungseinrichtung
- 590: Vorsprung
- 592: Vorsprung
- 610: Vorrichtung
- 612: Applikatorhülse
- 614: Endabschnitt
- 618: Austrageöffnung
- 620: Aktivierungseinrichtung
- 710: Vorrichtung
- 712: Applikatorhülse
- 714: Endabschnitt
- 718: Austrageöffnung
- 720: Aktivierungseinrichtung
- 738: Zapfen
- 740: Schlitz
- 790: Positionierelement
- 792: Positionierelement
- 810: Vorrichtung
- 812: Applikatorhülse
- 814: Endabschnitt
- 815: löffelförmiges Aufnahmegefäß
- 818: Austrageöffnung
- 820: Aktivierungseinrichtung
- 890: Positionierelement
- 892: Positionierelement
- 894: Positioniereinrichtung
- 910: Vorrichtung
- 912: Applikatorhülse
- 914: Endabschnitt
- 920: Aktivierungseinrichtung
- 932: Greifhilfe
- 952: Außenwand
- 954: Innenwand
- 956: Aktivierungselement
- 958: Positionierelement
- 960: Abschrägung
- 1010: Vorrichtung
- 1010': Vorrichtung
- 1010": Vorrichtung
- 1012: Applikatorhülse
- 1012': Applikatorhülse
- 1012": Applikatorhülse
- 1014: Endabschnitt
- 1014`: Endabschnitt
- 1014": Endabschnitt
- 1020: Aktivierungseinrichtung
- 1020': Aktivierungseinrichtung
- 1020": Aktivierungseinrichtung
- 1032: Greifhilfe
- 1038a: Zapfen
- 1038b: Zapfen
- 1038a`: Zapfen
- 1038b`: Zapfen
- 1038": Zapfen
- 1040a: Schlitz
- 1040b: Schlitz
- 1040a`: Schlitz
- 1040b`: Schlitz
- 1040": Schlitz
- 1054a: Teilhülse
- 1054b: Teilhülse
- 1054a`: Teilhülse
- 1054b`: Teilhülse
- 1054": Hülse
- 1056a: Aktivierungselement
- 1056b: Aktivierungselement
- 1056a`: Aktivierungselement
- 1056b`: Aktivierungselement
- 1056a": Aktivierungselement
- 1056b": Aktivierungselement
- 1058: Deckelelement

## Patentansprüche

1. Vorrichtung zum Austragen eines fließfähigen Stoffes, insbesondere zum Applizieren eines medizinischen Stoffes, eines pharmazeutischen Stoffes, eines Nahrungsergänzungsmittels oder eines Kosmetikums, umfassend eine Aktivierungseinrichtung (120, 120', 120", 120‴, 120"", 220, 220", 220‴, 320, 520, 620, 720, 820, 820', 920, 1020, 1020', 1020") und einen Körper (112, 112', 112", 112‴, 112" ", 212, 212', 212", 212‴ 312, 412, 412', 512, 612, 712, 812, 812', 912, 1012, 1012', 1012"), der an einem ersten Ende eine Austrageöffnung (118, 518, 618, 718) hat und einen Aufnahmeraum bildet, in dem eine zerbrechbare Kapsel (22, 22a, 22b) angeordnet ist, **dadurch gekennzeichnet, dass** die Kapsel (22, 22a, 22b) zwei Enden mit jeweils einer Spitze (24, 24a, 24b, 26, 26a, 26b) und jeweils einer Sollbruchstelle (25, 27) zum Entfernen der jeweiligen Spitze (24, 24a, 24b, 26, 26a, 26b) umfasst und durch Betätigung der Aktivierungseinrichtung (120, 120', 120", 120"', 120ʺʺ, 220, 220", 220'", 320, 520, 620, 720, 820, 820', 920, 1020, 1020', 1020") beide Spitzen (24, 24a, 24b, 26, 26a, 26b) an den betreffenden Sollbruchstellen (25, 27) abbrechbar sind, sodass der fließfähige Stoff ausbringbar ist.

2. Vorrichtung nach Anspruch 1, **dadurch gekennzeichnet, dass** die Kapsel (22, 22a, 22b) durch die Aktivierungseinrichtung (120, 120', 120", 120'", 120"", 220, 220", 220'", 320, 520, 620, 720, 820, 820', 920, 1020, 1020', 1020") und/oder den Körper (112, 112', 112", 112"', 112"", 212, 212', 212", 212'", 312, 412, 412', 512, 612, 712, 812, 812', 912, 1012, 1012', 1012") in dem Aufnahmeraum positioniert ist.

3. Vorrichtung nach Anspruch 1 oder 2, **dadurch gekennzeichnet, dass** der Körper an dem ersten Ende einen Endabschnitt (114, 114', 114", 114‴, 114"", 214', 214", 214 ` ` `, 514, 614, 714, 814, 814', 914, 1014, 1014', 1014") hat, in dem die erste Spitze (24, 24a, 24b) der Kapsel (22, 22a, 22b) angeordnet ist.

4. Vorrichtung nach Anspruch 3, **dadurch gekennzeichnet, dass** der Endabschnitt (114, 114', 114", 114‴, 114"", 214', 214", 214‴, 514, 614, 814, 1014, 1014', 1014") aus einem elastischen, brechbaren oder stauchbaren Material ist.

5. Vorrichtung nach Anspruch 3 oder 4, **dadurch gekennzeichnet, dass** der Endabschnitt (114ʺʺ, 214, 214', 214", 214'", 514, 614, 714) ein Kippverschluss oder ein Luer-System ist, der Endabschnitt (114, 114', 114", 114‴) Greifelemente (115) hat und/oder der Endabschnitt (814) an einem dem Körper abgewandten Ende einen Löffel ausbildet.

6. Vorrichtung nach einem der Ansprüche 1 bis 5, **dadurch gekennzeichnet, dass** die Aktivierungseinrichtung ein Kolben (220, 220', 220", 220‴, 920, 1020, 1020', 1020") ist oder aus einem flexiblen Material (120, 120', 120", 120‴, 120ʺʺ, 120"', 620, 820, 820'), insbesondere einer Gummikappe, oder als Vakuum-Kammer gebildet ist.

7. Vorrichtung nach Anspruch 6, **dadurch gekennzeichnet, dass** die Aktivierungseinrichtung (120, 120', 120", 120'", 120"", 220, 220", 220'", 520, 620, 720, 820, 820', 920, 1020, 1020', 1020") durch ein axiales Verschieben in Richtung der Kapsel (22, 22a, 22b), durch ein Umbiegen, durch eine Rotation und/oder durch Aufbringen eines Drucks betätigbar ist.

8. Vorrichtung nach Anspruch 7, **dadurch gekennzeichnet, dass** die Aktivierungseinrichtung (220', 220", 720, 920, 1020, 1020', 1020") einen Zapfen (238', 238", 738, 938, 1038, 1038', 1038") hat, der in einem an dem Körper (212', 212", 712, 912, 1012, 1012', 1012") ausgebildeten, mit dem Zapfen (238', 238", 738, 938, 1038, 1038', 1038") korrespondierenden Schlitz (240', 240", 740, 940, 1040, 1040', 1040") angeordnet ist, sodass ein Verschiebe- bzw. Rotationsweg der Aktivierungseinrichtung (220', 220", 720, 920, 1020, 1020', 1020") relativ zu dem Körper (212', 212", 712, 912, 1012, 1012', 1012") begrenzt ist.

9. Vorrichtung nach einem der Ansprüche 6 bis 8, **dadurch gekennzeichnet, dass** zwischen der Aktivierungseinrichtung (220, 220', 220", 220‴, 720) und dem Körper (212, 212', 212", 212'" 712) ein Rückstellelement ausgebildet ist, das die Aktivierungseinrichtung (220, 220', 220", 220‴, 720) nach einer Betätigung in deren Ausgangslage zurückbringt.

10. Vorrichtung nach einem der Ansprüche 1 bis 9, **gekennzeichnet durch** eine Greifhilfe (132, 132', 132", 132'", 132"", 232, 232', 232", 232'", 932, 1032).

11. Vorrichtung nach einem der Ansprüche 1 bis 10, **dadurch gekennzeichnet, dass** der Körper (112ʺʺ, 312, 412, 412', 612, 812, 812') einen elastischen Bereich hat, mittels dem der fließfähige Stoff definiert aus der Kapsel (22, 22a, 22b) ausbringbar ist.

12. Vorrichtung nach einem der Ansprüche 1 bis 5, **dadurch gekennzeichnet, dass** der Körper (312) eine Tube ist.

13. Vorrichtung nach Anspruch 12, **dadurch gekennzeichnet, dass** der Körper (312) eine Tube (312) und die Aktivierungseinrichtung eine Verschlussnaht (320), insbesondere eine Schweißnaht ist, die an dem der Austrageöffnung abgewandten Ende ausgebildet ist, wobei sich in der Verschlussnaht (320) die zweite Spitze (26) bzw. ein Teil der zweiten Spitze (26) der Kapsel (22) befindet und die Aktivierungseinrichtung durch ein Knicken betätigbar ist.

14. Vorrichtung nach einem der Ansprüche 1 bis 13, **dadurch gekennzeichnet, dass** der Körper (1012, 1012', 1012") mindestens zwei Aufnahmeräume ausbildet, in denen jeweils eine Kapsel (22a, 22b) angeordnet ist.

## Claims

1. A device for discharging a flowable substance, in particular for applying a medical substance, a pharmaceutical substance, a food supplement or a cosmetics, the device comprising an activation mechanism (120, 120', 120", 120‴, 120ʺʺ, 220, 220", 220‴, 320, 520, 620, 720, 820, 820', 920, 1020, 1020', 1020") and a body (112, 112', 112", 112‴, 112ʺʺ, 212, 212', 212", 212‴ 312, 412, 412', 512, 612, 712, 812, 812', 912, 1012, 1012', 1012") which has a discharge opening (118, 518, 618, 718) at a first end and forms a containing space in which a frangible capsule (22, 22a, 22b) is disposed, **characterized in that** the capsule (22, 22a, 22b) has two ends each having a tip (24, 24a, 24b, 26, 26a, 26b) and each having a predetermined breaking point (25, 27) for removing the respective tip (24, 24a, 24b, 26, 26a, 26b) and **in that** both tips (24, 24a, 24b, 26, 26a, 26b) can be snapped off at the respective predetermined breaking point (25, 27) by actuating the activation mechanism (120, 120', 120", 120‴, 120"", 220, 220", 220‴, 320, 520, 620, 720, 820, 820', 920, 1020, 1020', 1020") so that the flowable substance is discharged.

2. The device according to claim 1, **characterized in that** the capsule (22, 22a, 22b) is positioned in the containing space by the activation mechanism (120, 120', 120", 120‴, 120"", 220, 220", 220‴, 320, 520, 620, 720, 820, 820', 920, 1020, 1020', 1020") and/or the body (112, 112', 112", 112‴, 112"", 212, 212', 212", 212‴ 312, 412, 412', 512, 612, 712, 812, 812', 912, 1012, 1012', 1012").

3. The device according to claim 1 or 2, **characterized in that** the body has an end section (114, 114', 114", 114‴, 114"", 214', 214", 214‴, 514, 614, 714, 814, 814', 914, 1014, 1014', 1014") at the first end, the end section being where the first tip (24, 24a, 24b) of the capsule (22, 22a, 22b) is disposed.

4. The device according claim 3, **characterized in that** the end section (114, 114', 114", 114‴, 114"", 214', 214", 214‴, 514, 614, 814, 1014, 1014', 1014") is made of an elastic, frangible or compressible material.

5. The device according to claim 3 or 4, **characterized in that** the end section (114"", 214, 214', 214", 214‴, 514, 614, 714) is a tipping lock or a Luer lock, the end section (114, 114', 114", 114‴) has grip elements (115) and/or the end section (814) forms a spoon at an end facing away from the body.

6. The device according to one of the claims 1 to 5, **characterized in that** the activation mechanism is a piston (220, 220', 220", 220‴, 920, 1020, 1020', 1020") or is made of a flexible material (120, 120', 120", 120‴, 120"", 120‴, 620, 820, 820'), in particular a rubber cap, or is realized as a vacuum chamber.

7. The device according to claim 6, **characterized in that** the activation mechanism (120, 120', 120", 120‴, 120ʺʺ, 220, 220", 220‴, 520, 620, 720, 820, 820', 920, 1020, 1020', 1020") is actuated by being axially displaced, bent, rotated and/or exerting pressure in the direction of the capsule (22, 22a, 22b).

8. The device according to claim 7, **characterized in that** the activation mechanism (220', 220", 720, 920, 1020, 1020', 1020") has a pin (238', 238", 738, 938, 1038, 1038', 1038") which is disposed in a slit (240', 240", 740, 940, 1040, 1040', 1040") corresponding to the pin (238', 238", 738, 938, 1038, 1038', 1038") and formed on the body (212', 212", 712, 912, 1012, 1012', 1012") so that a displacement or rotation path of the activation mechanism (220', 220", 720, 920, 1020, 1020', 1020") is limited relative to the body (212', 212", 712, 912, 1012, 1012', 1012").

9. The device according to one of the claims 6 to 8, **characterized in that** a resetting element, which resets the activation mechanism (220, 220', 220", 220‴, 720) into its initial position after actuation, is realized between the activation mechanism (220, 220', 220", 220‴, 720) and the body (212, 212', 212", 212‴, 712).

10. The device according to one of the claims 1 to 9, **characterized by** a holding aid (132, 132', 132", 132‴, 132ʺʺ, 232, 232', 232", 232‴, 932, 1032).

11. The device according to one of the claims 1 to 10, **characterized in that** the body (112"", 312, 412, 412', 612, 812, 812') has an elastic area by means of which the flowable substance can be discharged from the capsule (22, 22a, 22b) in a defined manner.

12. The device according to one of the claims 1 to 5, **characterized in that** the body (312) is a tube.

13. The device according to claim 12, **characterized in that** the body (312) is a tube (312) and the activation mechanism is a seam (320), in particular a welding seal, which is formed on the end facing away from the discharge opening, the second tip (26) or a part of the second tip (26) of the capsule (22) being disposed in the seam (320) and the activation mechanism being actuated by being bent.

14. The device according to one of the claims 1 to 13, **characterized in that** the body (1012, 1012', 1012") forms at least two containing spaces in each of which one capsule (22a, 22b) is disposed.

## Revendications

1. Dispositif pour le décharge d'un substance fluide, notamment pour l'application d'un substance médical, d'un substance pharmaceutique, d'un complément alimentaire ou de cosmétiques, le dispositif comprenant un mécanisme d'activation (120, 120', 120", 120‴, 120ʺʺ, 220, 220", 220‴, 320, 520, 620, 720, 820, 820', 920, 1020, 1020', 1020") et un corps (112, 112', 112", 112‴, 112"", 212, 212', 212", 212‴ 312, 412, 412', 512, 612, 712, 812, 812', 912, 1012, 1012', 1012") qui a une ouverture de décharge (118, 518, 618, 718) à une première extrémité et forme un espace de conteneur dans lequel une capsule frangible (22, 22a, 22b) est disposée, **caractérisé en ce que** la capsule (22, 22a, 22b) a deux extrémités chacun ayant un bout (24, 24a, 24b, 26, 26a, 26b) et chacun ayant un point de rupture (25, 27) pour enlever le bout (24, 24a, 24b, 26, 26a, 26b) respectif et **en ce que** les deux bouts (24, 24a, 24b, 26, 26a, 26b) peuvent être arrachés au point de rupture (25, 27) respectif en activant le mécanisme d'activation (120, 120', 120", 120‴, 120"", 220, 220", 220‴, 320, 520, 620, 720, 820, 820', 920, 1020, 1020', 1020") de manière à ce que le substance fluide est déchargé.

2. Dispositif selon la revendication, **caractérisé en ce que** la capsule (22, 22a, 22b) est positionnée dans l'espace de conteneur par le mécanisme d'activation (120, 120', 120", 120‴, 120"", 220, 220", 220‴, 320, 520, 620, 720, 820, 820', 920, 1020, 1020', 1020") et/ou par le corps (112, 112', 112", 112‴, 112ʺʺ, 212, 212', 212", 212‴ 312, 412, 412', 512, 612, 712, 812, 812', 912, 1012, 1012', 1012").

3. Dispositif selon la revendication 1 ou la revendication 2, **caractérisé en ce que** le corps a une zone d'extrémité (114, 114', 114", 114‴, 114"", 214', 214", 214‴, 514, 614, 714, 814, 814', 914, 1014, 1014', 1014") à la première extrémité, la zone d'extrémité étant l'endroit où le premier bout (24, 24a, 24b) de la capsule (22, 22a, 22b) est disposé.

4. Dispositif selon la revendication 3, **caractérisé en ce que** la zone d'extrémité (114, 114', 114", 114‴, 114ʺʺ, 214', 214", 214‴, 514, 614, 814, 1014, 1014', 1014") est fait d'un matériau élastique, frangible ou compressible.

5. Dispositif selon la revendication 3 ou la revendication 4, **caractérisé en ce que** la zone d'extrémité (114"", 214, 214', 214", 214‴, 514, 614, 714) est une fermeture basculante ou une fermeture Luer, la zone d'extrémité (114, 114', 114", 114‴) a des éléments de prise (115) et/ou la zone d'extrémité (814) forme une cuillère à une extrémité tournée le dos au corps.

6. Dispositif selon l'une quelconque des revendications 1 à 5, **caractérisé en ce que** le mécanisme d'activation est un piston (220, 220', 220", 220‴, 920, 1020, 1020', 1020") ou est fait d'un matériau flexible (120, 120', 120", 120‴, 120ʺʺ, 120‴, 620, 820, 820'), notamment d'un capuchon en caoutchouc, ou est réalisé comme chambre sous vide.

7. Dispositif selon la revendication 6, **caractérisé en ce que** le mécanisme d'activation (120, 120', 120", 120‴, 120"", 220, 220", 220‴, 520, 620, 720, 820, 820', 920, 1020, 1020', 1020") est activé en étant déplacé, plié, tourné axialement et/ou en exerçant de la pression en sens de la capsule (22, 22a, 22b).

8. Dispositif selon la revendication 7, **caractérisé en ce que** le mécanisme d'activation (220', 220", 720, 920, 1020, 1020', 1020") a une goupille (238', 238", 738, 938, 1038, 1038', 1038") qui est disposé dans une fente (240', 240", 740, 940, 1040, 1040', 1040") correspondante à la goupille (238', 238", 738, 938, 1038, 1038', 1038") et formée sur le corps (212', 212", 712, 912, 1012, 1012', 1012") de manière à ce qu'un trajet de déplacement ou de rotation du mécanisme d'activation (220', 220", 720, 920, 1020, 1020', 1020") est limité par rapport au corps (212', 212", 712, 912, 1012, 1012', 1012").

9. Dispositif selon l'une quelconque des revendications 6 à 8, **caractérisé en ce qu'**un élément de rappel, qui rappelle le mécanisme d'activation (220, 220', 220", 220‴, 720) dans sa position initiale après l'activation, est réalisé entre le mécanisme d'activation (220, 220', 220", 220‴, 720) et le corps (212, 212', 212", 212‴, 712).

10. Dispositif selon l'une quelconque des revendications 1 à 9, **caractérisé par** une aide de prise (132, 132', 132", 132‴, 132"", 232, 232', 232", 232‴, 932, 1032).

11. Dispositif selon l'une quelconque des revendications 1 à 10, **caractérisé en ce que** le corps (112"", 312, 412, 412', 612, 812, 812') a une zone élastique au moyen de laquelle la substance fluide peut être déchargée à partir de la capsule (22, 22a, 22b) dans une manière définie.

12. Dispositif selon l'une quelconque des revendications 1 à 5, **caractérisé en ce que** le corps (312) est un conduit.

13. Dispositif selon la revendication 12, **caractérisé en ce que** le corps (312) est un conduit (312) et le mécanisme d'activation est un joint (320), notamment un cordon de soudure, qui est formé sur l'extrémité tournant le dos à l'ouverture de décharge, le deuxième bout (26) ou une partie du deuxième bout (26) de la capsule (22) étant disposé dans le joint (320) et le mécanisme d'activation pouvant être activé en étant plié.

14. Dispositif selon l'une quelconque des revendications 1 à 13, **caractérisé en ce que** le corps (1012, 1012', 1012") forme au moins deux espaces de conteneur dans chacune de laquelle une capsule (22a, 22b) est disposée.
